# EUROPEAN PATENT APPLICATION

(11) **EP 2 700 444 A1**
(43) Date of publication of application: **26.02.2014**
(21) Application number: 12827093.1
(22) Date of filing: 10.05.2012
(51) Int. Cl.: B01J 19/12, C12M 3/00, G01N 22/00

(54) **REACTION DEVICE**

(30) Priority: 02.09.2011 JP 2011191207
(71) Applicant: Tabuse, Katsuyoshi, Wakayama 640-8472 (JP); Sunny Engineering Co., Ltd, Hirano-ku, Osaka-city Osaka 547-0014 (JP)
(72) Inventor: ONCHI, Minoru, Osaka-city Osaka 547-0014 (JP)
(74) Representative: HOFFMANN EITLE
(86) International application number: PCT/JP2012/062056
(87) International publication number: WO 2013/031293

(57) **Abstract**

[Problem] The purpose of the invention is to provide a reaction device that, when reacting multiple samples simultaneously, can react the multiple samples substantially uniformly.

[Solution] The reaction device comprises: a microwave oscillation means for generating microwaves; holding containers (24) for individually holding multiple samples collected from human bodies, etc.; a microwave irradiation container (20) on which each of the holding containers (24) can be individually loaded; a temperature sensor (23) for detecting the temperature of a sample held in a holding container (24) or of the interior of the microwave irradiation container (20); and a microwave control means for varying the microwaves generated by the microwave oscillation means on the basis of the temperature detected by the temperature sensor (23). The microwave irradiation container (20) comprises: a microwave introduction port (22) for introducing the microwaves generated by the microwave oscillation means into the microwave irradiation container (20); and ring-shaped patterns (21f) for irradiating each of the holding containers (24) with the microwaves introduced from the microwave introduction port (22).

## Description

### Technical Field

The present invention relates to a reaction device for reacting samples, such as cell culture.

### Background Art

Chemical reaction devices through the use of a microwave have conventionally been used in the field of chemical reactions, biochemical reactions, etc., when a trace amount of chemical synthesis, decomposition reactions, DNA analyses, etc., are carried out by using samples having been collected from human bodies etc., (for example, Patent Document 1).

### Prior Art Document

### Patent Document

Patent Document 1: Japanese Published Unexamined Patent Application Publication No. 2009-154138

### Summary of the Invention

### Problems to be Solved by the Invention

Although chemical reaction devices using microwaves have conventionally been used, conventional chemical reaction devices can react only one sample, so the devices are significantly inefficient when a plurality of samples are desired to be reacted since the samples have to be reacted one by one.

In addition, to react a plurality of samples simultaneously, a microwave to be irradiated to each of the samples needs to be adjusted to react the samples substantially uniformly. Therefore, there is also a problem of involving technical difficulties in reacting a plurality of samples simultaneously.

Accordingly, in view of the foregoing circumstances, the present invention aims to provide a reaction device capable of reacting a plurality of samples substantially uniformly when reacting the samples simultaneously.

### Means for Solving the Problems

The foregoing object of the present invention will be achieved by the following means. It is noted that numerals and alphabetic letters in parentheses are reference codes of embodiments described later but the present invention should not be limited to them.

A reaction device according to claim 1 of the present invention includes a microwave oscillation means (a microwave oscillating section 30a) to oscillate a microwave, holding containers (24, 52) correspondingly holding a plurality of samples having been collected from human bodies etc., a microwave irradiation container (20) in which each of the holding containers (24, 52) can be individually placed, a temperature sensor (23) for detecting the temperature of the sample held in the holding container (24, 52) or the temperature within the microwave irradiation container (20), and a microwave control means (a microwave control section 31a) to vary the microwave oscillated by the microwave oscillation means (the microwave oscillating section 30a) on the basis of the temperature having been detected by the temperature sensor (23). The reaction device is characterized in that the microwave irradiation container (20) has a microwave introduction port (22) which introduces the microwave having been oscillated by the microwave oscillation means (the microwave oscillating section 30a) into the microwave irradiation container (20), and microwave irradiation means (ring patterns 21 f, rectangular patch antennas 51 e) to correspondingly irradiate the holding containers (24, 52) with the microwave having been introduced from the microwave introduction port (22).

While a reaction device according to claim 2 includes a microwave oscillation means (a microwave oscillating section 30a) to oscillate a microwave, holding containers (125) correspondingly holding a plurality of samples having been collected from human bodies etc., a microwave irradiation container (120, 210) in which each of the holding containers (125) can be individually placed, a temperature sensor (126) for detecting the temperature of the sample held in the holding container (125) or the temperature within the microwave irradiation container (120, 210), and a microwave control means (a microwave control section 31 a) to vary the microwave oscillated by the microwave oscillation means (the microwave oscillating section 30a) on the basis of the temperature having been detected by the temperature sensor (126). The reaction device is characterized in that the microwave irradiation container (120, 210) has a microwave introduction port (122, 212) which introduces the microwave having been oscillated by the microwave oscillation means (the microwave oscillating section 30a) into the microwave irradiation container (120, 210) and a microwave irradiation means (a coaxial central conductor 123, 213) to irradiate the holding containers (125) with the microwave having been introduced from the microwave introduction port (122, 212), and the holding containers (125) are placed in such a manner so as to surround the periphery of the microwave irradiation means (the coaxial central conductor 123, 213).

The invention of claim 3 is characterized by, in the reaction device as set forth in claim 1 or 2, an electric power monitoring means (an electric power monitoring section 30c) to receive a reflected wave of the microwave from the microwave introduction port (22, 122, 212) and to determine whether the received reflected wave exceeds a predetermined value, wherein on an occasion when the electric power monitoring means (the electric power monitoring section 30c) determines that the received reflected wave exceeds the predetermined value, the microwave control means (the microwave control section 31a) stops the microwave oscillated by the microwave oscillation means (the microwave oscillating section 30a).

The invention of claim 4 is characterized in that, in the reaction device as set forth in claim 1 or 2, the microwave irradiation container (20, 120, 210) is placed within a constant temperature bath (4).

Further, a reaction device according to the invention of claim 5 includes holding containers (52) correspondingly holding a plurality of samples having been collected from human bodies etc., a microwave irradiation container (20) in which each of the holding containers (52) can be individually placed, and a microwave oscillation means (a microwave oscillating section 300a) to oscillate a predetermined microwave at every predetermined time interval. The reaction device is characterized in that the microwave irradiation container (20) has a microwave introduction port (22) which introduces the microwave having been oscillated by the microwave oscillation means (the microwave oscillating section 300a) into the microwave irradiation container (20), and microwave irradiation means (rectangular patch antennas 51e) to correspondingly irradiate the holding containers (52) with the microwave having been introduced from the microwave introduction port (22).

### Effects of the Invention

Next, effects of the present invention will be described attaching reference numerals to the drawings. First, in the reaction device according to the invention of claim 1, the holding containers (24, 52) correspondingly holding a plurality of samples are individually placed in the microwave irradiation container (20), and the temperature of the sample held in the holding container (24, 52) or the temperature within the microwave irradiation container (20) is detected by the temperature sensor (23). The detected temperature is output to the microwave control means (the microwave control section 31a), and this microwave control means (the microwave control section 31 a) varies the microwave oscillated by the microwave oscillation means (the microwave oscillating section 30a) on the basis of the above temperature. The varied microwave is output, via the microwave oscillation means (the microwave oscillating section 30a), to the microwave introduction port (22) which introduces the microwave into the microwave irradiation container (20). In addition, the microwave introduced from the microwave introduction port (22) is irradiated to each of the holding containers (24, 52) by the corresponding microwave irradiation means (the ring patterns 21f, the rectangular patch antennas 51 e). As a result, a substantially uniform microwave can be irradiated to the plurality of samples, so that reactions of these samples can be kept substantially uniform.

On the other hand, in the reaction device according to the invention of claim 2, the holding containers (125) correspondingly holding a plurality of samples are individually placed in the microwave irradiation container (120, 210), and the temperature of the sample held in the holding container (125) or the temperature within the microwave irradiation container (120, 210) is detected by the temperature sensor (126). The detected temperature is output to the microwave control means (the microwave control section 31a), and this microwave control means (the microwave control section 31a) varies the microwave oscillated by the microwave oscillation means (the microwave oscillating section 30a) on the basis of the above temperature. The varied microwave is output, via the microwave oscillation means (the microwave oscillating section 30a), to the microwave introduction port (122, 212) which introduces the microwave into the microwave irradiation container (120, 210). In addition, the microwave introduced from the microwave introduction port (122, 212) is irradiated to those holding containers (125) by the microwave irradiation means (the coaxial central conductor 123, 213). Since these holding containers (125) are placed in such a manner so as to surround the periphery of the microwave irradiation means (the coaxial central conductor 123, 213), a substantially uniform microwave can be irradiated to the plurality of samples. As a result, reactions of these samples can be kept substantially uniform.

Further, according to the invention of claim 3, the electric power monitoring means (the electric power monitoring section 30c) receives a reflected wave of the microwave from the microwave introduction port (22, 122, 212) and determines whether the received reflected wave exceeds a predetermined value. On the occasion when the electric power monitoring means (the electric power monitoring section 30c) determines that the received reflected wave exceeds the predetermined value, the microwave control means (the microwave control section 31a) stops the microwave oscillated by the microwave oscillation means (the microwave oscillating section 30a). As a result, the control over the microwave oscillation means so as not to oscillate an anomalous microwave can be done, and thus, breakage of the microwave oscillation means can be reduced.

Further, according to the invention of claim 4, reactions of the samples having been collected from human bodies etc. and held within the holding containers (24, 52, 125) can be made more favorable by placing the microwave irradiation container (20, 120, 210) within the constant temperature bath (4).

On the other hand, in the reaction device according to the invention of claim 5, the holding containers (52) correspondingly holding a plurality of samples are individually placed in the microwave irradiation container (20), and a predetermined microwave is introduced into the microwave irradiation container (20) via the microwave oscillation means (the microwave oscillating section 300a) at every predetermined time interval. The microwave introduced from the microwave introduction port (22) is irradiated to each of the holding containers (52) by the corresponding microwave irradiation means (the rectangular patch antennas 51e). As a result, a substantially uniform microwave can be irradiated to the plurality of samples, so that reactions of these samples can be kept substantially uniform.

### Brief Description of the Drawings

FIG. 1 is a diagram showing a plan view of a reaction device according to the first embodiment of the present invention, in which an applicator according to the reaction device is illustrated in a cross-sectional view.
FIG. 2 is a front view of the applicator according to the same embodiment.
FIG. 3 is a block diagram of a microwave oscillation control unit according to the same embodiment.
FIG. 4 is a diagram showing a plan view of a reaction device according to the second embodiment of the present invention, in which an applicator according to the reaction device is shown in a cross-sectional view.
FIG. 5 is a front view of the applicator according to the same embodiment.
FIG. 6 is a diagram showing a front view of a reaction device according to the third embodiment of the present invention, in which an applicator according to the reaction device is shown in a longitudinal sectional view.
FIG. 7A is a plan view of a cover body of the applicator according to the same embodiment.
FIG. 7B is a sectional view taken along line X-X of FIG. 6.
FIG. 8 is a diagram showing a front view of a reaction device according to the fourth embodiment of the present invention, in which an applicator according to the reaction device is shown in a longitudinal sectional view.
FIG. 9A is a plan view of a cover body of the applicator according to the same embodiment.
FIG. 9B is a sectional view taken along line Y-Y of FIG. 8.
FIG. 10 is a diagram showing a plan view of a reaction device according to the fifth embodiment of the present invention, in which an applicator according to the reaction device is shown in a cross-sectional view.
FIG. 11 is a front view of the applicator according to the same embodiment.
FIG. 12 is a block diagram of a microwave oscillation control unit according to the same embodiment.

### Modes for Carrying Out the Invention

### <First embodiment>

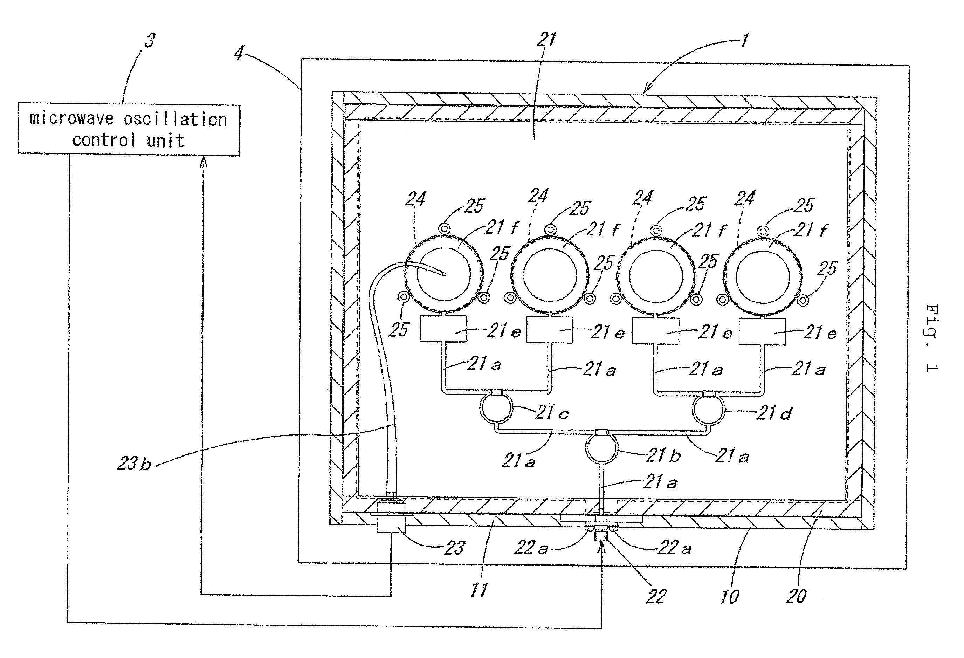

Hereinafter, the first embodiment according to the present invention will be described in detail with reference to FIGS. 1 to 3.

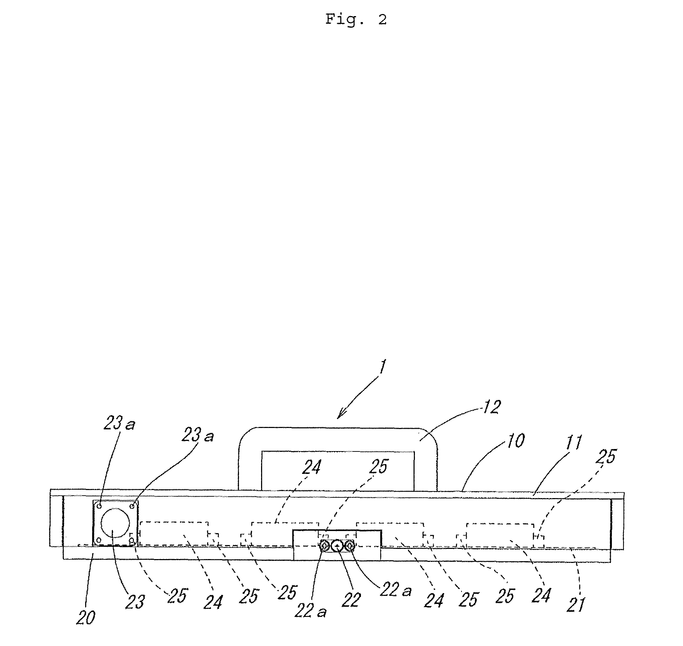

As shown in FIG. 1, a reaction device according to the present embodiment is constituted of an applicator 1, a microwave oscillation control unit 3 supplying the applicator 1 with a microwave, and a constant temperature bath 4 capable of keeping its internal temperature constant. The applicator 1 is constituted of a cover body 10 formed of aluminum etc., and a rectangular parallelepiped microwave irradiation container 20 formed such that the top is opened and the interior is hollow, and also the peripheral edge is slightly thick-walled, as shown in FIG. 1 and FIG. 2. The thus constituted applicator 1 is placed within the constant temperature bath 4, as shown in FIG. 1.

As shown in FIG. 2, the cover body 10 is constituted of a cover main body 11 being U-shaped when viewed from the front and a grip portion 12 fixed on a top portion of the cover main body 11 by welding etc. More specifically, the cover main body 11 is formed so as to close the top of the microwave irradiation container 20 and to surround a peripheral frame of the microwave irradiation container 20, as shown in FIG. 1 and FIG. 2. The grip portion 12 allows the cover main body 11 to be moved so as to be separated from the microwave irradiation container 20. As a result, the top of the microwave irradiation container 20 can be opened and closed freely. In the present embodiment, an example of the cover body 10 and the microwave irradiation container 20 being separable is given. However, the cover body 10 and the microwave irradiation container 20 may be formed integrally by using a hinge etc.

Meanwhile, the microwave irradiation container 20 is formed of aluminum etc., and as shown in FIG. 1 and FIG. 2, a rectangular printed circuit board 21 is fixed in the interior thereof by screws etc. (not shown). The microwave irradiation container 20 is provided with a microwave introduction port 22 composed of a SMA coaxial connector fixed at a substantially central portion of a lateral surface by screws 22a, and also provided with a contact type temperature sensor 23 fixed at an end portion of the lateral surface by screws 23a. From this microwave introduction port 22, the microwave having been output from the microwave oscillation control unit 3 is supplied into the microwave irradiation container 20. In the present embodiment, a SMA coaxial connector is given as an example of the microwave introduction port 22. However, an N coaxial connector may be used, as a matter of course.

A microwave introduction passage 21a formed so as to be conducted with the microwave introduction port 22 is patterned on the printed circuit board 21. The patterned microwave introduction passage 21a is divided into two by a distributor 21b, and the two-way microwave introduction passage 21a is further divided into four by distributors 21c and 21d. The distributors 21b, 21c, and 21d are patterned on the printed circuit board 21, and use of such distributors can reduce reflected waves of microwaves.

The microwave introduction passage 21a divided into four as described above is connected to corresponding ring patterns 21f via corresponding matching boxes 21e for performing impedance matching. These matching boxes 21e and ring patterns 21f are also patterned on the printed circuit board 21.

Meanwhile, holding containers 24 composed of Petri dishes holding samples having been collected from human bodies etc., are placed on the corresponding ring patterns 21f. As shown in FIG. 1, the outer diameter of the ring patterns 21f is formed slightly larger than that of the holding containers 24. A microwave can be irradiated to the holding containers 24 effectively by forming the outer diameter of the ring patterns 21f slightly larger than that of the holding containers 24 as just described. That is, if the outer diameter of the ring pattern 21 f is made somewhere around the center of the holding container 24, a circumferential region thereof is not irradiated. If the ring pattern 21 f is formed to surround the outer diameter of the holding container 24, a contact region between the ring pattern 21f and the holding container 24 itself disappears. Therefore, this leads to significantly inefficient irradiation. Further, in order to position and fix the holding container 24 on the ring pattern 21 f, as shown in FIG. 1 and FIG. 2, cylindrical holding members 25 are arranged, on the printed circuit board 21, at necessary places (three places in the drawings) of the circumferential frame of the ring pattern 21f.

Further, the temperature sensor 23 is attached with a sheathed thermocouple wire 23b in the interior of the microwave irradiation container 20 as shown in FIG. 1. A distal end of the sheathed thermocouple wire 23b is introduced into one holding container 24, and the temperature of a sample held within that holding container 24 is detected. The detected temperature is output to the microwave oscillation control unit 3 by the temperature sensor 23 via the sheathed thermocouple wire 23b as shown in FIG. 1. The reason why the distal end of the sheathed thermocouple wire 23b is introduced into one holding container 24 is that a microwave under the same conditions is irradiated to the holding container 24 and the other holding containers 24 (three containers in the drawing) as described above, so that as long as the distal end of the sheathed thermocouple wire 23b is introduced into one holding container 24, the temperatures of the samples held within the corresponding holding containers 24 can all be managed.

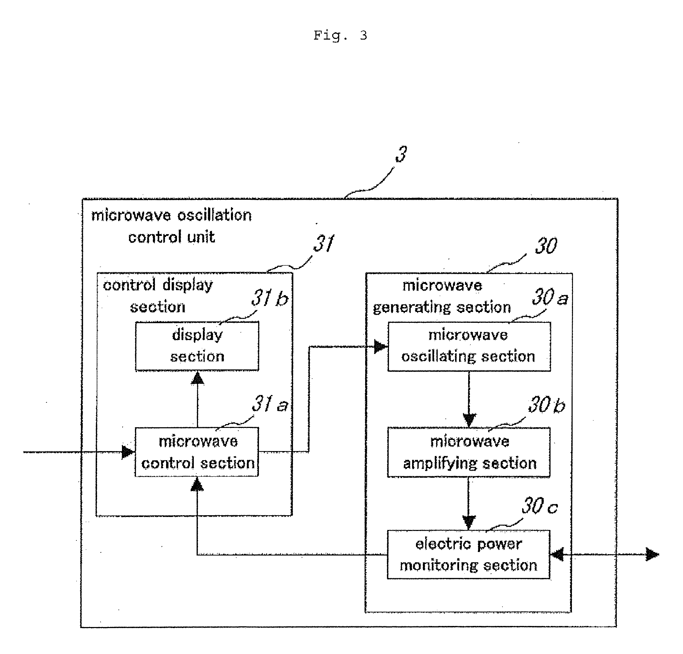

Meanwhile, the microwave oscillation control unit 3 is constituted of a microwave generating section 30 and a control display section 31, as shown in FIG. 3. The microwave generating section 30 is constituted of a microwave oscillating section 30a, a microwave amplifying section 30b, and an electric power monitoring section 30c. The microwave oscillating section 30a is capable of oscillating a microwave and stopping the oscillation based on commands from the control display section 31. Meanwhile, the microwave oscillated by the microwave oscillating section 30a as just described is amplified by the microwave amplifying section 30b and output to the electric power monitoring section 30c. The electric power monitoring section 30c having received the output outputs the amplified microwave to the microwave introduction port 22, and also receives a reflected wave having been output from the microwave introduction port 22. On the occasion when the received reflected wave exceeds a predetermined value, the electric power monitoring section 30c outputs a command to stop the oscillation of the microwave to the control display section 31. As a result, the control over the microwave oscillating section 30a and the microwave amplifying section 30b so as not to oscillate an anomalous microwave can be done, and thus, breakage of the microwave oscillating section 30a and the microwave amplifying section 30b can be reduced.

The control display section 31 is constituted of a microwave control section 31 a and a display section 31b. The microwave control section 31a receives the temperature of the sample held within the holding container 24 having been detected by the temperature sensor 23, and varies the microwave substantially continuously in order to keep the sample temperature constant, and outputs a command, to the microwave oscillating section 30a, to oscillate a microwave continuing in point of time (a microwave being non-intermittent in point of time (a microwave in which a time when the output becomes 0 does not continue)). By this, the microwave oscillating section 30a oscillates a microwave based on the command.

In addition, on the occasion when the microwave control section 31 a receives a command to stop the oscillation of the microwave having been output from the electric power monitoring section 30c, the microwave control section 31a outputs a command to stop the oscillation of the microwave to the microwave oscillating section 30a. With this, the microwave oscillating section 30a stops the oscillation of the microwave. Further, the display section 31 b is composed of a liquid crystal etc., and can display the temperature having been detected by the temperature sensor 23 or can display a stop signal of the microwave, via the microwave control section 31a.

The microwave introduced into the applicator 1 is controlled by the microwave oscillation control unit 3 as described above. Therefore, by placing the applicator 1 inside the constant temperature bath 4 capable of keeping its internal temperature constant, reactions of the samples having been collected from human bodies etc., and held within the holding containers 24 can be made more favorable. That is, on the occasion when the temperature within the constant temperature bath 4 is set at 37 degrees C. for example, the temperature of the samples held within the holding containers 24 does not become a constant temperature due to the relationship with the irradiation of the microwave, and changes to temperatures around 37 degrees C. Therefore, the microwave control section 31a varies the microwave substantially continuously so as to keep the temperature having been detected by the temperature sensor 23 constant, and continues to output the command to oscillate a microwave continuous in point of time to the microwave oscillating section 30a. As a result, the microwave oscillating section 30a continues to oscillate a microwave based on the command, and the microwave introduced into the applicator 1 through the use of the microwave oscillation control unit 3 is irradiated to the holding containers 24 without a pause. Consequently, reactions of the samples held within the holding containers 24 can be made more favorable.

According to the present embodiment, the holding containers 24 correspondingly holding a plurality of samples are placed on the microwave irradiation container 20, and the temperature of the sample held within the holding container 24 is detected by the temperature sensor 23. The detected temperature is output to the microwave control section 31a, and this microwave control section 31a varies the microwave oscillated by the microwave oscillating section 30a on the basis of the above temperature. The varied microwave is output, via the microwave oscillating section 30a, to the microwave introduction port 22 which introduces the microwave into the microwave irradiation container 20. In addition, the microwave introduced from the microwave introduction port 22 is then irradiated to the holding containers 24 correspondingly by the ring patterns 21f. As a result, a substantially uniform microwave is irradiated to the plurality of samples, whereupon reactions of these samples can be kept substantially uniform.

Further, it is preferable to use Petri dishes as the holding containers in the present embodiment. An example of use of the samples held within the holding containers includes cell culture.

### <Second Embodiment>

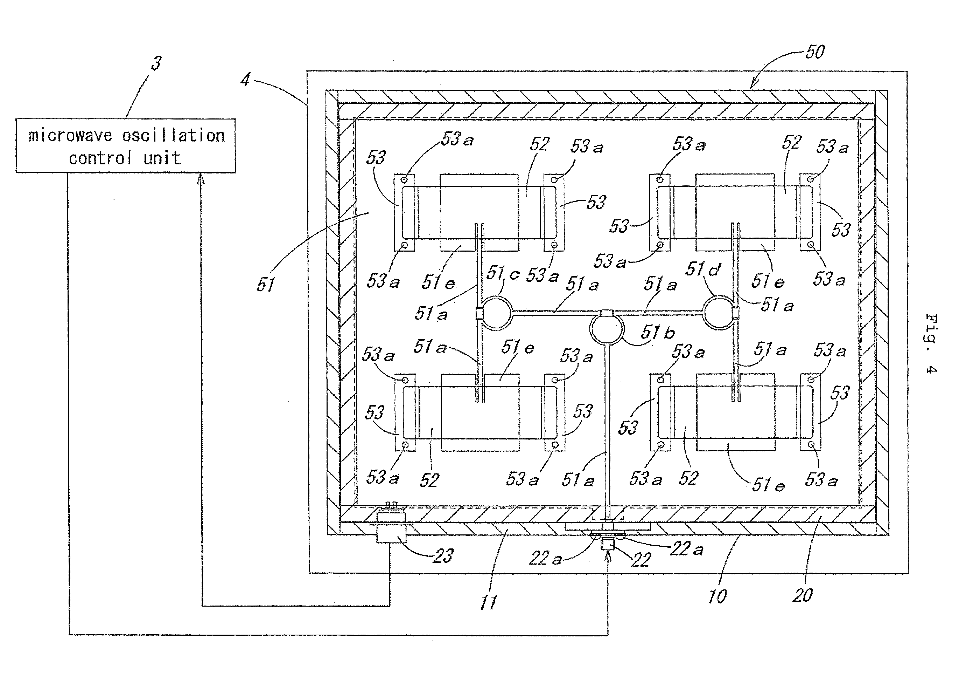

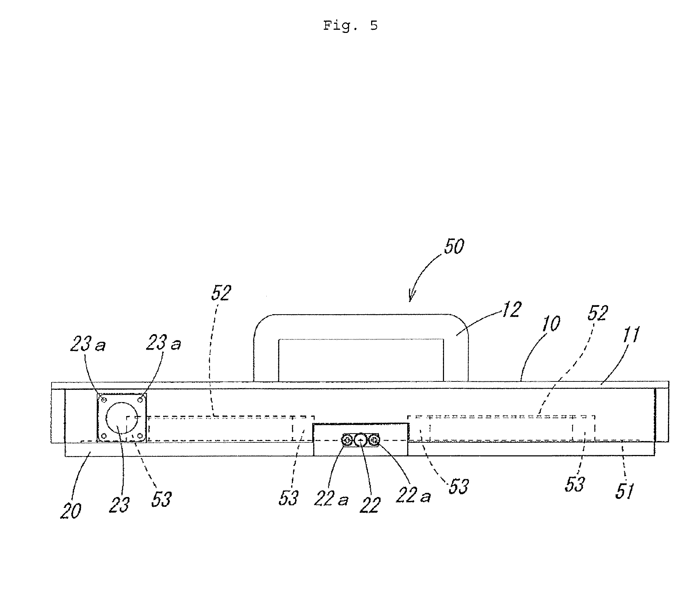

Next, the second embodiment according to the present invention will be described in detail with reference to FIG. 4 and FIG. 5. The same configurations as those of the first embodiment will be given the same numerals, and descriptions thereof will be omitted.

As shown in FIG. 4, a reaction device according to the present embodiment is constituted of an applicator 50, a microwave oscillation control unit 3 supplying the applicator 50 with a microwave, and a constant temperature bath 4 capable of keeping its internal temperature constant. As shown in FIG. 4 and FIG. 5, the applicator 50 is constituted of a cover body 10 and a microwave irradiation container 20. As shown in FIG. 4, the thus constituted applicator 50 is placed within the constant temperature bath 4. As shown in FIG. 4 and FIG. 5, a rectangular printed circuit board 51 is fixed in the interior of the microwave irradiation container 20 by screws etc. (not shown). The microwave irradiation container 20 is provided with a microwave introduction port 22 composed of a SMA coaxial connector fixed at a substantially central portion of a lateral surface by screws 22a, and also provided with a contact type temperature sensor 23 fixed at an end portion of the lateral surface by screws 23a. From this microwave introduction port 22, the microwave having been output from the microwave oscillation control unit 3 is supplied into the microwave irradiation container 20.

A microwave introduction passage 51a formed so as to be conducted with the microwave introduction port 22 is patterned on the printed circuit board 51. The patterned microwave introduction passage 51a is divided into two by a distributor 51b, and the two-way microwave introduction passage 51a is further divided into four by distributors 51 c and 51d. The distributors 51 b, 51 c, and 51d are patterned on the printed circuit board 51, and use of such distributors can reduce reflected waves of microwaves.

The microwave introduction passage 51a divided into four as described above is connected to corresponding rectangular patch antennas 51e. Further, these rectangular patch antennas 51e are also patterned on the printed circuit board 51.

Meanwhile, holding containers 52 composed of preparations holding samples having been collected from human bodies etc., of a diameter slightly smaller than the width of the patch antennas 51e are correspondingly arranged on the rectangular patch antennas 51e. Accordingly, the microwave is irradiated to each of the holding containers 52 by the corresponding patch antennas 51e. Further, in order to position and fix the holding container 52 on the rectangular patch antenna 51e, as shown in FIG. 4 and FIG. 5, a pair of substantially rectangular parallelepiped holding members 53 are arranged, on the printed circuit board 51, at both lateral sides of each rectangular patch antenna 51e. On these holding members 53, the holding container 52 is placed. Each holding member 53 is fixed on the printed circuit board 51 by screws 53a as shown in FIG. 4.

On the other hand, the temperature sensor 23 is different from the first embodiment, and detects the temperature within the microwave irradiation container 20 and outputs the detected temperature to the microwave oscillation control unit 3 as shown in FIG. 4. This is because an amount of sample that the holding container 52 composed of a preparation can hold is very small and a very large difference between the temperature within the microwave irradiation container 20 and the temperature of the sample held by the holding container 52 does not occur.

According to the present embodiment, however, the holding containers 52 correspondingly holding a plurality of samples are individually placed on the microwave irradiation container 20, and the temperature within the microwave irradiation container 20 is detected by the temperature sensor 23. The detected temperature is output to the microwave control section 31a, and this microwave control section 31 a varies the microwave oscillated by the microwave oscillating section 30a on the basis of the above temperature. The varied microwave is output, via the microwave oscillating section 30a, to the microwave introduction port 22 which introduces the microwave into the microwave irradiation container 20. In addition, the microwave introduced from the microwave introduction port 22 is then irradiated to each of the holding containers 52 by the corresponding rectangular patch antennas 51e. As a result, a substantially uniform microwave is irradiated to the plurality of samples, whereupon reactions of these samples can be kept substantially uniform.

In the present embodiment, it is preferable to use preparations as the holding containers. An example of use of the samples held within the holding containers includes a fluorescent antibody technique.

### <Third Embodiment>

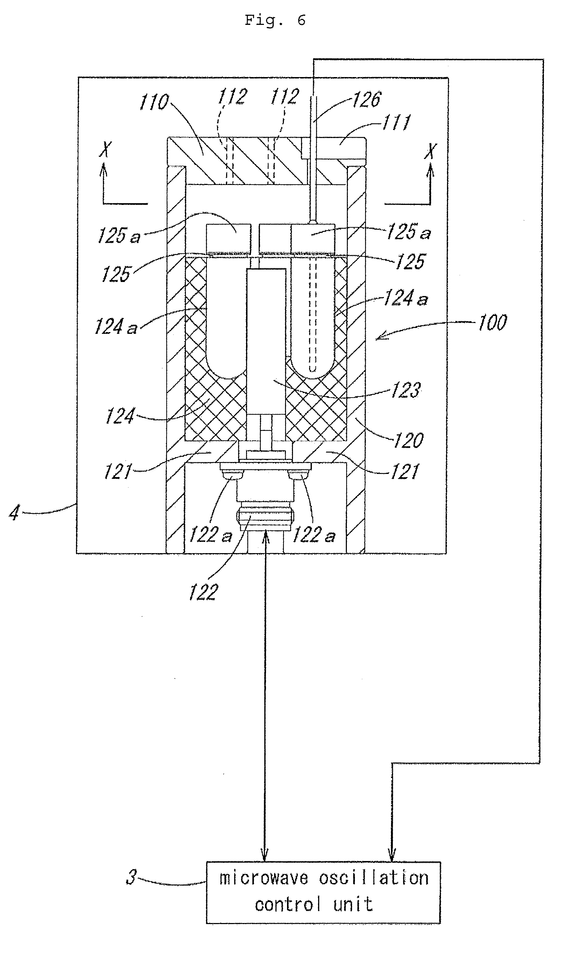

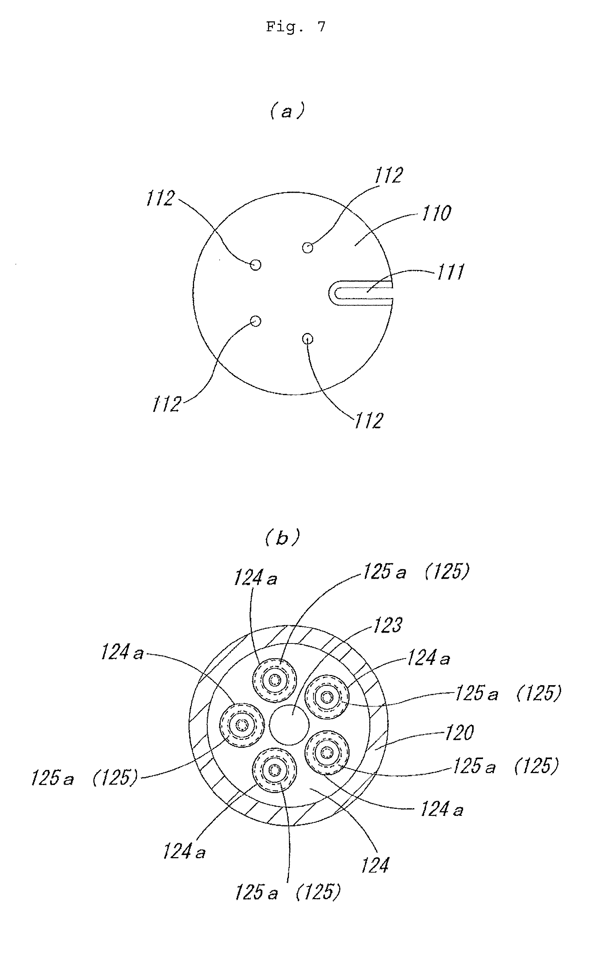

Subsequently, the third embodiment of the present invention will be described in detail with reference to FIG. 6 and FIGs. 7 (a) - (b). The same configurations as those of the first embodiment will be given the same numerals, and descriptions thereof will be omitted.

As shown in FIG. 6, a reaction device according to the present embodiment is constituted of an applicator 100, a microwave oscillation control unit 3 supplying the applicator 100 with a microwave, and a constant temperature bath 4 capable of keeping its internal temperature constant. As shown in FIG. 6, the applicator 100 is constituted of a cover body 110 formed of aluminum etc., and a substantially cylindrical microwave irradiation container 120 whose top and bottom are opened. The thus constituted applicator 100 is placed within the constant temperature bath 4, as shown in FIG. 6.

The cover body 110 is formed substantially in the shape of a circle in a plan view as shown in FIG. 7(a), and formed in a thick plate shape as shown in FIG. 6. The diameter of the cover body 110 is formed so as to close the top of the microwave irradiation container 120 as shown in FIG. 6, and the cover body 110 is configured to be freely separable from the microwave irradiation container 120.

Further, as shown in FIG. 6 and FIG. 7(a), the cover body 110 has one end portion formed with a substantially oval long hole 111 for introducing a temperature sensor 126 composed of a thermocouple which will be described later into the microwave irradiation container 120. At necessary places (four places in the drawings (see FIG. 7(a)), steam vents 112 for discharging steam generated within the microwave irradiation container 120 to the outside are provided. In the present embodiment, an example of the cover body 110 and the microwave irradiation container 120 being separable is given. However, the cover body 110 and the microwave irradiation container 120 may be formed integrally by using a hinge etc.

Meanwhile, the microwave irradiation container 120 is formed of aluminum etc., and as shown in FIG. 6, formed in a substantially cylindrical shape whose top and bottom are opened, and at the bottom, a doughnut-shaped mounting base 121 is projectingly integrally provided. As shown in FIG. 6, the mounting base 121 has an undersurface provided with a microwave introduction port 122 composed of an N coaxial connector fixed on the undersurface of the mounting base 121 by screws 122a. A coaxial central conductor 123 irradiated with the microwave having been output from the microwave oscillation control unit 3 is attached at one end side of the microwave introduction port 122 (the upper surface side of the mounting base 121).

Further, a holding container storage base 124 formed of Teflon (registered mark) etc., having high microwave permeability is arranged within the microwave irradiation container 120 and at the upper surface side of the mounting base 121 in such a manner so as to surround the circumference of the coaxial central conductor 123. As shown in FIG. 6 and FIG. 7(b), substantially semi-oval storage holes 124a capable of storing holding containers 125 composed of test tubes whose tops are closed by caps 125a and holding samples having been collected from human bodies etc., are provided in the holding container storage base 124 at predetermined intervals in such a manner so as to surround the circumference of the coaxial central conductor 123.

As described above, the holding containers 125 holding samples having been collected from human bodies etc., are stored in corresponding storage holes 124a provided in the holding container storage base 124. The thus stored holding containers 125 are stored in such a manner so as to surround the coaxial central conductor 123. Therefore, the microwave irradiated from the coaxial central conductor 123 is irradiated substantially uniformly to the holding containers 125. Further, the holding container storage base 124 is freely separable from inside the microwave irradiation container 120, and the holding containers 125 are also stored so as to be separable from the holding container storage base 124.

Further, a temperature sensor 126 composed of a thermocouple introduced from the long hole 111 of the cover body 110 is introduced into one of the holding containers 125 and detects the temperature of the sample held within the holding container 125. The temperature sensor 126 then outputs the detected temperature to the microwave oscillation control unit 3 as shown in FIG. 6. The reason why the temperature sensor 126 is introduced into one holding container 125 is that a microwave under the same conditions is irradiated to the holding container 125 and the other holding containers 125 (four containers in the drawings) as described earlier, so that as long as the temperature sensor 126 is introduced into one holding container 125, the temperatures of the samples correspondingly held within the holding containers 125 can all be managed. In the present embodiment, the temperature of the sample held in the holding container 125 is detected by using the temperature sensor 126, but the temperature within the microwave irradiation container 120 may be detected. However, it is preferable, in order to improve accuracy, to detect the temperature of the sample held in the holding container 125.

According to the present embodiment, however, the holding containers 125 correspondingly holding a plurality of samples are individually placed in the microwave irradiation container 120, and the temperature of the sample held within the holding container 125 is detected by the temperature sensor 126. The detected temperature is output to the microwave control section 31a, and this microwave control section 31a varies the microwave oscillated by the microwave oscillating section 30a on the basis of the above temperature. The varied microwave is output, via the microwave oscillating section 30a, to the microwave introduction port 122 which introduces the microwave into the microwave irradiation container 120. The microwave introduced from the microwave introduction port 122 is then irradiated to the holding containers 125 by the coaxial central conductor 123. Since these holding containers 125 are placed in such a manner so as to surround the circumference of the coaxial central conductor 123, a substantially uniform microwave can be irradiated to the plurality of samples, whereupon reactions of these samples can be kept substantially uniform.

Further, it is preferable to use test tubes as the holding containers in the present embodiment. However, preparations etc., may be used. On that occasion, each storage hole 124a provided in the holding container storage base 124 has only to be provided in a hole shape adapted to the shape of the preparation etc. Further, an example of use of the samples held within the holding containers includes inorganic and organic reactions, cell culture.

### <Fourth Embodiment>

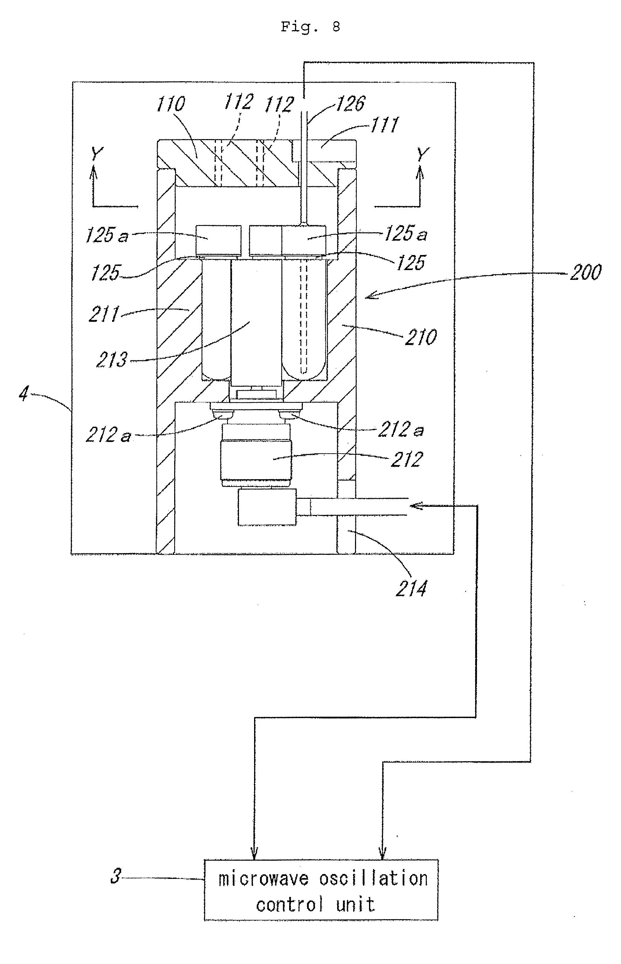

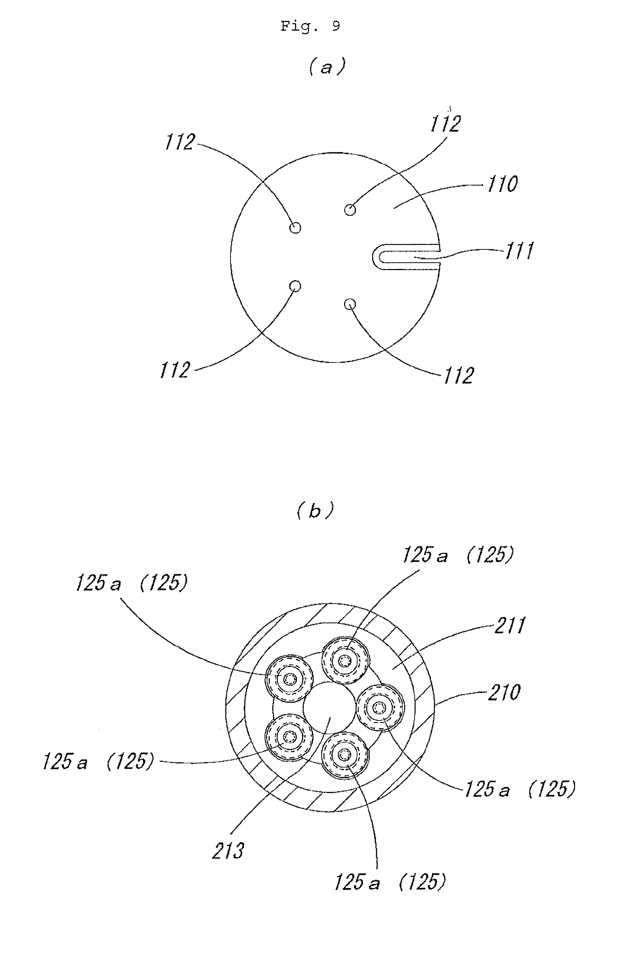

Next, the fourth embodiment of the present invention will be described in detail with reference to FIG. 8 and FIGs. 9 (a) - (b). The same configurations as those of the first and third embodiments will be given the same numerals, and descriptions thereof will be omitted.

As shown in FIG. 8, a reaction device according to the present embodiment is constituted of an applicator 200, a microwave oscillation control unit 3 supplying the applicator 200 with a microwave, and a constant temperature bath 4 capable of keeping its internal temperature constant. As shown in FIG. 8, the applicator 200 is constituted of a cover body 110 formed of aluminum etc., and a substantially cylindrical microwave irradiation container 210 whose top and bottom are opened. The thus constituted applicator 200 is placed within the constant temperature bath 4, as shown in FIG. 8.

The microwave irradiation container 210 is formed of aluminum etc., having high thermal conductivity, and as shown in FIG. 8, formed in a substantially cylindrical shape whose top and bottom are opened. From the middle toward the top thereof, a holding container storage base 211 is projectingly integrally provided. As shown in FIG. 9(b), the thus provided holding container storage base 211 has an inner circumferential edge formed with circular arcs at predetermined intervals such that holding containers 125 (see FIG. 8) composed of test tubes whose tops are closed by caps 125a and holding samples having been collected from human bodies etc., can be stored on the circumference. As shown in FIG. 8, the holding container storage base 211 has an undersurface provided with a microwave introduction port 212 composed of an N coaxial connector fixed on the undersurface of the holding container storage base 211 by screws 212a and bent at a right angle. At one end side of the microwave introduction port 212 (the upper side of the microwave irradiation container 210), a coaxial central conductor 213 irradiated with the microwave having been output from the microwave oscillation control unit 3 is attached. As a result, the holding containers 125 are stored in such a manner so as to surround the circumference of the coaxial central conductor 213. Further, an insertion hole 214 for inserting the microwave introduction port 212 into the microwave irradiation container 210 is provided at a lower portion of the microwave irradiation container 210. The holding containers 125 are stored so as to be freely separable from the holding container storage base 211.

According to the present embodiment, however, the holding containers 125 correspondingly holding a plurality of samples are individually placed in the microwave irradiation container 210, and the temperature of the sample held within the holding container 125 is detected by the temperature sensor 126. The detected temperature is output to the microwave control section 31a, and this microwave control section 31a varies the microwave oscillated by the microwave oscillating section 30a on the basis of the above temperature. The varied microwave is output, via the microwave oscillating section 30a, to the microwave introduction port 212 which introduces the microwave into the microwave irradiation container 120. The microwave introduced from the microwave introduction port 212 is then irradiated to the holding containers 125 by the coaxial central conductor 213. Since these holding containers 125 are placed in such a manner so as to surround the circumference of the coaxial central conductor 213, a substantially uniform microwave can be irradiated to the plurality of samples, whereupon reactions of these samples can be kept substantially uniform.

Further, it is preferable to use test tubes as the holding containers in the present embodiment. However, differing from the third embodiment, Teflon (registered mark) is not used in the present embodiment, because aluminum etc., having high thermal conductivity is used to efficiently cool down the holding containers. Accordingly, an example of use of the samples held within the holding containers is preferably for cell cryopreservation.

### <Fifth Embodiment>

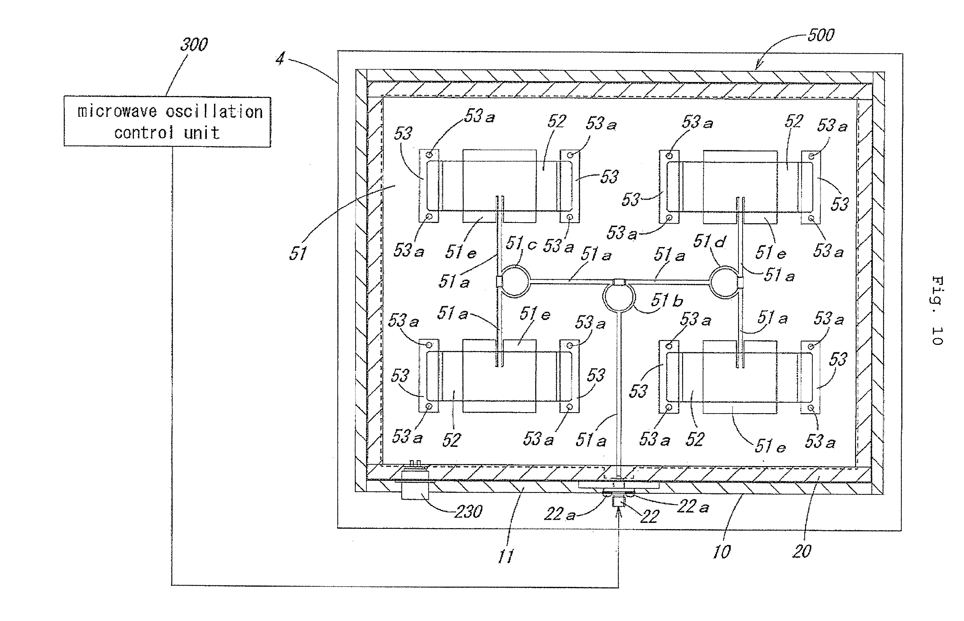

Next, the fifth embodiment of the present invention will be described in detail with reference to FIGS. 10 to 12. The same configurations as those of the first and second embodiments will be given the same numerals, and descriptions thereof will be omitted.

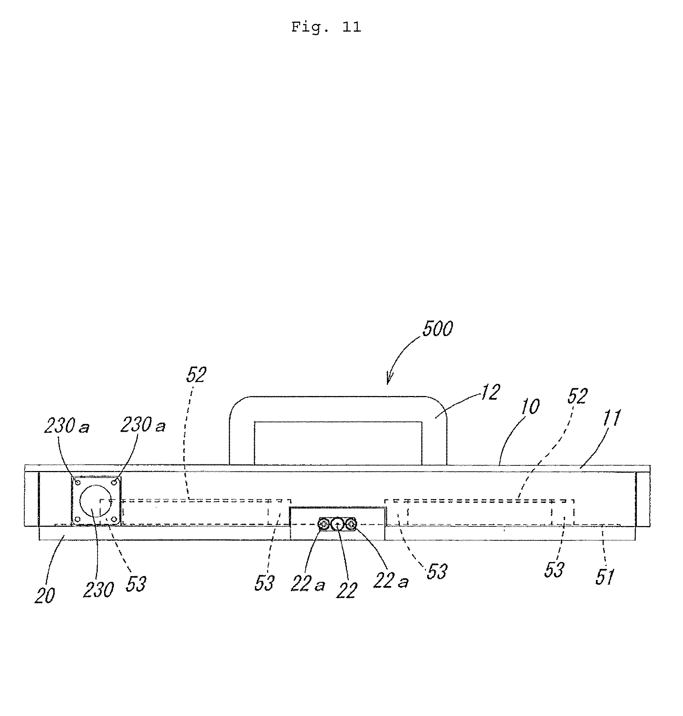

As shown in FIG. 10, a reaction device according to the present embodiment is constituted of an applicator 500, a microwave oscillation control unit 300 supplying the applicator 500 with a microwave, and a constant temperature bath 4 capable of keeping its internal temperature constant. As shown in FIG. 10 and FIG. 11, the applicator 500 is constituted of a cover body 10 and a microwave irradiation container 20. As shown in FIG. 10, the thus constituted applicator 500 is placed within the constant temperature bath 4. As shown in FIG. 10 and FIG. 11, a rectangular printed circuit board 51 is fixed in the interior of the microwave irradiation container 20 by screws etc. (not shown). The microwave irradiation container 20 is provided with a microwave introduction port 22 composed of a SMA coaxial connector fixed at a substantially central portion of a lateral surface by screws 22a, and also provided with a contact type temperature sensor 230 fixed at an end portion of the lateral surface by screws 230a. From this microwave introduction port 22, the microwave having been output from the microwave oscillation control unit 300 is supplied into the microwave irradiation container 20. The temperature sensor 230 is different from the first and second embodiments, and merely detects the temperature within the microwave irradiation container 20 and has nothing to do with the microwave oscillation control unit 300.

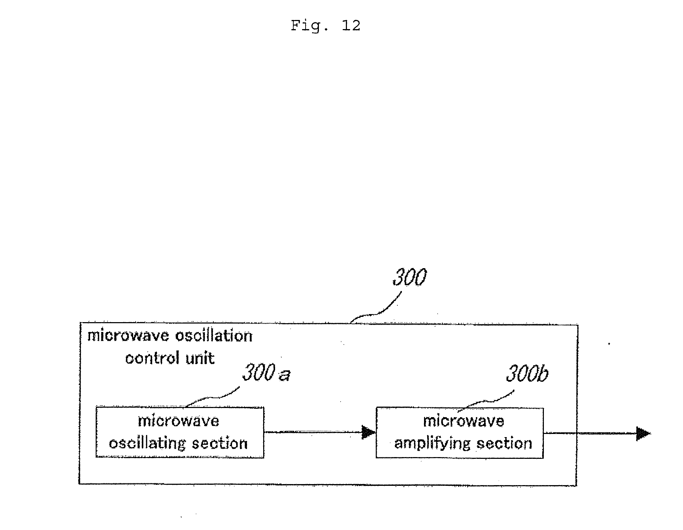

Meanwhile, the microwave oscillation control unit 300 is constituted of a microwave oscillating section 300a and a microwave amplifying section 300b as shown in FIG. 12. The microwave oscillating section 300a oscillates a predetermined microwave at every predetermined time interval. The microwave oscillated by the microwave oscillating section 300a as just described is amplified by the microwave amplifying section 300b and output to the microwave introduction port 22. As a result, the predetermined microwave is supplied into the microwave irradiation container 20 at every predetermined time interval.

According to the present embodiment, however, the holding containers 52 correspondingly holding a plurality of samples are individually placed on the microwave irradiation container 20, and the predetermined microwave is introduced into the microwave irradiation container 20 at every predetermined time interval via the microwave oscillating section 300a. The microwave introduced from the microwave introduction port 22 is then irradiated to each of the holding containers 52 by the corresponding rectangular patch antennas 51e. As a result, a substantially uniform microwave is irradiated to the plurality of samples, whereupon reactions of these samples can be kept substantially uniform.

Further, it is preferable to use preparations as the holding containers in the present embodiment. An example of use of the samples held within the holding containers includes a fluorescent antibody technique.

### Brief Description of the Numerals

1, 50, 100, 200, 500: Applicator
3, 300: Microwave oscillation control unit
4: Constant temperature bath
20, 120, 210: Microwave irradiation container
21b, 21c, 21d: Distributor
21f: Ring pattern (microwave irradiation means)
22, 122, 212: Microwave introduction port
23, 126: Temperature sensor
24, 52, 125: Holding container
30a: Microwave oscillating section (microwave oscillation means)
30c: Electric power monitoring section (electric power monitoring means)
31a: Microwave control section (microwave control means)
51 b, 51 c, 51d: Distributor
51e: Rectangular patch antenna (microwave irradiation means)
123, 213: Coaxial central conductor (microwave irradiation means)
300a: Microwave oscillating section (microwave oscillation means)

## Claims

1. A reaction device comprising
a microwave oscillation means to oscillate a microwave,
a microwave irradiation container,
a plurality of holding containers within the microwave irradiation container for holding a corresponding plurality of samples collected from human bodies,
a temperature sensor for detecting a temperature of at least one of the plurality of samples held in the holding containers or a temperature within the microwave irradiation container,
a microwave control means to vary the microwave oscillated by the microwave oscillation means on the basis of the temperature detected by the temperature sensor, the reaction device being **characterized in that**
the microwave irradiation container having a microwave introduction port which introduces the microwave oscillated by the microwave oscillation means into the microwave irradiation container, and microwave irradiation means to correspondingly irradiate the plurality of holding containers with the microwave having been introduced from the microwave introduction port.

2. A reaction device comprising:
a microwave oscillation means to oscillate a microwave,
a microwave irradiation container,
a plurality of holding containers within the microwave irradiation container, the plurality of holding containers holding a corresponding plurality of samples having been collected from human bodies,
a temperature sensor for detecting a temperature of at least one of the plurality of samples held in the holding containers or a temperature within the microwave irradiation container,
a microwave control means to vary the microwave oscillated by the microwave oscillation means on the basis of the temperature detected by the temperature sensor, the reaction device being **characterized in that**
the microwave irradiation container having a microwave introduction port which introduces the microwave having been oscillated by the microwave oscillation means into the microwave irradiation container and a microwave irradiation means to irradiate the plurality of holding containers with the microwave having been introduced from the microwave introduction port; and
the holding containers being placed in such a manner so as to surround a periphery of the microwave irradiation means.

3. The reaction device according to claim 1 or 2, further comprising:
an electric power monitoring means operable to receive a reflected wave of the microwave from the microwave introduction port and to determine whether the received reflected wave exceeds a predetermined value, the reaction device being **characterized in that**
the microwave control means being operable to stop the microwave oscillated by the microwave oscillation means when the electric power monitoring means determines that the received reflected wave exceeds the predetermined value.

4. The reaction device according to claim 1 or 2, **characterized in that** the microwave irradiation container being within a constant temperature bath.

5. A reaction device comprising:
a microwave irradiation container,
a plurality of holding containers within the microwave irradiation container, the plurality of holding containers correspondingly holding a plurality of samples collected from human bodies,
a microwave oscillation means to oscillate a predetermined microwave at every predetermined time interval, the reaction device being **characterized in that**
the microwave irradiation container having a microwave introduction port which introduces the microwave having been oscillated by the microwave oscillation means into the microwave irradiation container, and microwave irradiation means to correspondingly irradiate the holding containers with the microwave having been introduced from the microwave introduction port.
